(19) Europäisches Patentamt
European Patent Office
Office européen des brevets

(11) Numéro de publication : 0 349 431 B1

(12) FASCICULE DE BREVET EUROPEEN

(45) Date de publication du fascicule du brevet : **15.04.92 Bulletin 92/16**

(51) Int. Cl.$^5$ : **C07H 15/04,** A61K 31/70, C07H 5/02

(21) Numéro de dépôt : **89401860.5**

(22) Date de dépôt : **29.06.89**

# (54) Nouveaux produits dérivés du saccharose.

(30) Priorité : **29.06.88 FR 8808723**

(43) Date de publication de la demande : **03.01.90 Bulletin 90/01**

(45) Mention de la délivrance du brevet : **15.04.92 Bulletin 92/16**

(84) Etats contractants désignés : **BE CH DE ES FR GB IT LI NL**

(56) Documents cités :
**CAN. J. CHEM., vol. 37, 1959, pages 2047-2052; A.K. MITRA et al.: "The reaction of sucrose with glycol-cleaving oxidants"**
**TETRAHEDRON LETTERS, vol. 28, no. 8, 1987, pages 891-894, Pergamon Journals Ltd, GB; K.J. HALE et al.: "The cyclisation of the DI- and tetra-aldehydes derived from sucrose with nitroalkanes"**
**COMPTES RENDUS HEBDOMADAIRES DES SEANCES DE L'ACADEMIE DES SCIENCES, janvier-juin 1942, tome deux cent-quatorzième, pages 366-368, Gauthier-Villars, Imprimeur-Libraire, Paris, FR; P. FLEURY et al.: "L'oxidation du saccharose par l'acide periodique"**

(56) Documents cités :
**THE JOURNAL OF THE AMERICAN CHEMICAL SOCIETY, vol. 72, mai-août 1950, pages 2130-2132, Mack Pringting Co., Easton, PA, US; R.C. HOCKETT et al.: "Lead tetraacetate oxidations in the sugar group. XI. The oxidation of sucrose and preparation of glycerol and glycol"**

(73) Titulaire : **BEGHIN-SAY SOCIETE ANONYME**
**F-59239 Thumeries (FR)**

(72) Inventeur : **Badel, Agnès**
**46, rue Pierre Brunier**
**F-69300 Caluire (FR)**
Inventeur : **Descotes, Gérard**
**60, rue Waldeck Rousseau**
**F-69006 Lyon (FR)**
Inventeur : **Mentech, Julio**
**1d, rue Phelypeaux**
**F-69100 Villeurbanne (FR)**
Inventeur : **Thiriet, Bernard**
**42, avenue de la Terasse**
**F-91260 Juvisy (FR)**

(74) Mandataire : **Dorland, Anne-Marie et al**
**BEGHIN-SAY Service Propriété Industrielle 23 boulevard Georges Clemenceau**
**F-92402 Courbevoie Cédex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue Saint-Denis, 75001 PARIS

## Description

La présente invention concerne des nouveaux produits dérivés du saccharose, plus particulièrement des nouveaux produits d'oxydation de dérivés substitués du saccharose, ainsi que des procédés de préparation et des applications de ces produits.

Les hydrates de carbone comportant au moins un groupement glycol-1,2 sont potentiellement oxydables par l'ion periodate. Cette coupure oxydante pouvant être représentée par le schéma réactionnel

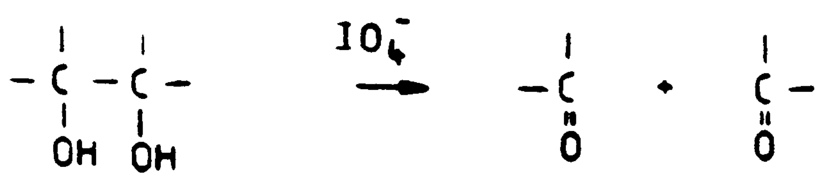

a été découverte par L. MALAPRADE, C.R., Acad. Sci., 1928, 186, 382. Dans le cas du saccharose, on obtient un produit connu dans la littérature sous le terme d'oxysaccharose et cité pour la première fois par P. FLEURY, J. COURTOIS et M. JAVILLIER, Acad. Sci. C.R., 1942, 214, 366. L'oxysaccharose peut également être obtenu par l'oxydation du saccharose au moyen de tétraacétate de plomb (R.C. HOCKETT et M. ZIEF, J. Am. Chem. Soc, 1950, 72 , 2130).

L'oxydation sélective du saccharose par du tétraacétate de plomb conduit au 3′,4′-dialdéhydosaccharose (voir Tétrahedron Letters, vol. 28, n° 8, 1987, p. 891-894). Il est également fait mention de ce produit dans la publication de A.K. MITRA et al., Can. J. Chem., vol. 37, 1959, p. 2047-2052, dans laquelle il est en outre indiqué qu'alors qu'en présence d'un excès de périodate ou de tétraacétate de plomb le saccharose est normalement oxydé pour donner de l'oxysaccharose, en présence d'une proportion moindre de tétraacétate de plomb on obtient une coupure au niveau du groupement glycol 3-4 de l'unité D-fructofuranosyle pour donner le polyol correspondant et qu'inversement le périodate de sodium n'attaque l'unité D-fructofuranosyle encore moins facilement que l'unité D-glucopyranosyle.

Les nouveaux produits de l'invention peuvent être représentés par la formule générale (I)

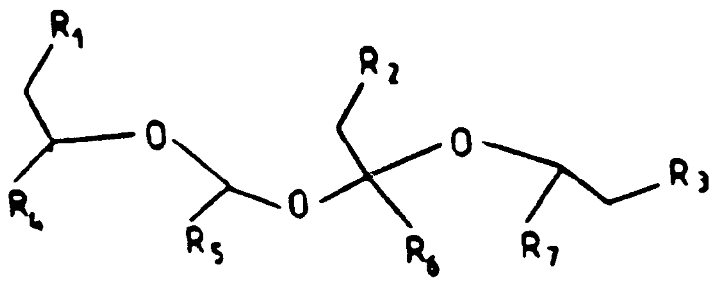

(I)

dans laquelle :

(a) les groupements $R_1$, $R_2$, et $R_3$ choisis parmi les radicaux X, OH, OR et OCOR′, où X est F, Cl, Br ou I, R est un groupement hydrocarboné contenant de 1 à 30 atomes de carbone et de 0 à 10 atomes de silicium et R′ est un groupement hydrocarboné contenant de 1 à 30 atomes de carbone,

(b) les groupements $R_4$ et $R_5$, identiques ou différents, sont choisis parmi les radicaux CHO, HOCH-CHO, ou bien $R_4$-$R_5$ est CHOH-CHOH-CHOH (configuration glucose),

(c) les groupements $R_6$ et $R_7$ sont CHO ou bien $R_6$-$R_7$ est CHOH-CHOH (configuration fructose), et dans laquelle

(i) on n'a pas en même temps $R_4$-$R_5$ = CHOH-CHOH-CHOH (configuration glucose) et $R_6$-$R_7$ = CHOH-CHOH (configuration fructose)

et

(ii) on n'a pas en même temps $R_1 = R_2 = R_3$ = OH.

Comme exemples de groupements R on peut citer les groupements alkyle, alkylaryle, aryle, alkylsilyle, arylsilyle, alkylarylsilyle contenant jusqu'à 30 atomes de carbone et jusqu'à 10 atomes de silicium.

Comme exemples de groupement R′ on peut citer les groupements alkyle, alcényle et alcynyle contenant

jusqu'à 30 atomes de carbone.

Dans un mode de réalisation particulier de l'invention, les composés de formule générale (I) ci-dessus sont tels que X = Cl ; R est choisi parmi les radicaux Tr, TBDMS et TBDPS ; où

– Tr représente le radical :

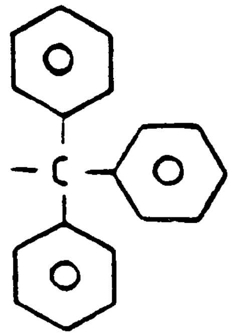

– TBDMS représente le radical tert-butyldiméthylsilyle de formule :

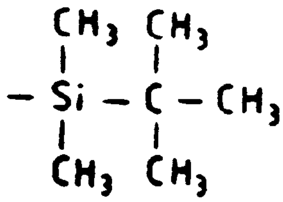

– TBDPS représente le radical tert-butyldiphénylsilyle de formule :

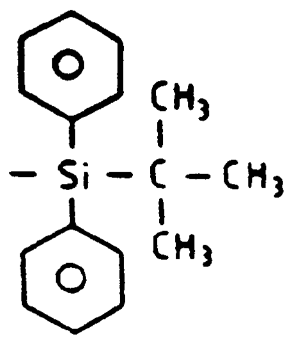

et R' est choisi parmi les groupements $C_nH_{2n+1}$ où n est un nombre entier pouvant aller de 1 à 30.

Les nouveaux produits représentés par la formule générale (I) ci-dessus peuvent être obtenus, selon la présente invention, par coupure oxydante de dérivés substitués du saccharose pouvant être représentés par la formule générale (II)

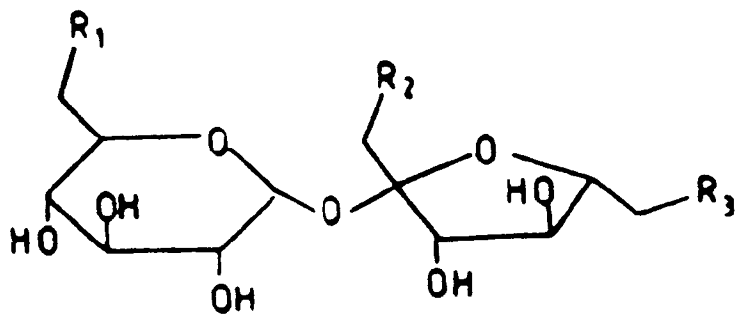

(II)

dans laquelle $R_1$, $R_2$ et $R_3$ ont la même signification que celle indiquée précédemment en présence de l'ion periodate, par exemple au moyen de l'acide periodique ou d'un de ses sels tel un sel de métal alcalin ou d'un periodate fixé sur résine, alumine ou silice, ou au moyen d'un réactif équivalent tel le tétraacétate de plomb conduisant à la coupure d'α-diols. Le choix de la nature et de la position des groupements substituants dudit dérivé substitué du saccharose de départ permet d'obtenir, selon la présente invention, une régiosélectivité de ladite coupure en une, deux ou trois positions, comme cela sera expliqué en détails ci-après.

Les produits d'oxydation obtenus sont caractérisés par leurs produits de réduction et d'acétylation.

Selon un procédé de la présente invention, on ajoute au dérivé substitué du saccharose de départ dissous dans un solvant approprié, choisi parmi l'eau et les solvants organiques tels par exemple le chloroforme ou le dichlorométhane, une solution aqueuse contenant l'ion periodate en quantité telle que le rapport molaire du periodate audit dérivé substitué du saccharose de départ soit compris entre 1 et 50, de préférence entre 4 et 20. Il peut être nécessaire d'incorporer un agent de transfert de phase au mélange obtenu pour catalyser la réaction dans le cas où celle-ci est trop lente. Ledit agent de transfert peut par exemple être un ammonium quaternaire tel l'Aliquat 336 (marque de fabrique de General Mills, Inc) ou tout autre catalyseur de transfert de phase. Le mélange réactionnel est d'abord maintenu à une température comprise entre -20 et 80°C, de préférence entre 0 et 20°C. On laisse la réaction se poursuivre pendant une durée suffisante, comprise entre environ 2 et 48 heures, en particulier de 2 à 14 heures. Dans le cas où la réaction est effectuée en milieu aqueux, on neutralise le mélange réactionnel avec une base, par exemple avec $NaHCO_3$ avant de précipiter les sels inorganiques par addition d'un solvant tel l'éthanol ou le propanol. Après précipitation, on filtre les produits et les filtrats sont évaporés.

Dans le cas où la réaction est effectuée en milieu biphasique, la phase organique est évaporée.

Selon un autre procédé de la présente invention, on ajoute au dérivé substitué du saccharose du départ dissous dans un solvant organique approprié tel par exemple de chloroforme ou la pyridine, du tétraacétate de plomb ($Pb(OAc)_4$), en quantité telle que le rapport molaire du tétraacétate de plomb au dérivé substitué du saccharose de départ soit au moins égal à 1 de préférence au moins égal à 4. On maintient le mélange réactionnel à une température comprise entre -20 et 80°C de préférence à température ambiante pendant 2 à 48 heures, en particulier 2 à 14 heures.

Après addition d'un acide tel l'acide oxalique, filtration, neutralisation et lavage, la phase organique est évaporée.

Les produits obtenus par les procédés décrits ci-dessus peuvent être caractérisés par réduction et acétylation. La réduction est effectuée sur les produits oxydés, dissous dans une solution alcoolique, par exemple éthanol ou butanol, par addition d'un agent réducteur tel le borohydrure de sodium (ajouté à raison de 4 moles par mole de produit à réduire). Après agitation pendant environ 12 heures à température ambiante, une solution d'acide, par exemple une solution aqueuse d'acide acétique (5 % V/V) ou une résine acide est ajoutée jusqu'à l'arrêt de production de gaz. Après filtration et évaporation en présence de méthanol, l'acétylation est effectuée en milieu pyridinique en ajoutant de l'acide acétique (20 moles par mole de produit) à une tempréature voisine de 0°C. Après 12 H d'agitation à température ambiante, l'évaporation azéotropique (toluène) fournit le mélange à chromatographier. Comme cela a été mentionné plus haut, la présente invention permet, par le choix approprié des groupements substituants du dérivé substitué du saccharose de départ, d'obtenir, avec des conditions opératoires appropriées, trois catégories de produits au moyen des procédés décrits précédemment. On peut par exemple obtenir :

– une monocoupure régiosélective de l'unité glucopyranosidique dans les cas suivants :

$R_1 = R_2 = R_3 = OTr$ (coupure orientée sur la liaison C-2, C-3)

ou

$R_1 = R_2 = R_3 = OTBDMS$ (coupure orientée sur la liaison C-3, C-4)

– une double coupure sélective de l'unité glucopyranosidique dans les cas suivants :

$R_1 = R_3 = Cl$ et $R_2$ = OTBDMS ou OTr
ou
$R_1 = R_2 = R_3$ = OTBDMS, OTr ou OTBDPS
ou
$R_1 = R_3$ = OTr ou OTBDMS et $R_2$ = OH
ou
$R_1$ = OTr et $R_2 = R_3$ = OH

– une coupure en trois positions glycol-1,2 présentes sur la molécule du dérivé substitué de saccharose de départ dans les cas suivants :
$R_1 = R_2 = R_3 = Cl$
ou
$R_1 = R_2 = R_3$ = OTr
ou
$R_1 = R_3$ = OTBDMS et $R_2$ = OH
ou
$R_1 = R_3 = OCOC_nH_{2n+1}$ et $R_2$ = OH
ou
$R_1 = R_2 = R_3$ = OH ou $OCOC_nH_{2n+1}$

Dans certains des cas mentionnés ci-dessus à titre d'exemple, il est possible d'obtenir, avec les même groupements substitués du dérivé du saccharose de départ, une monocoupure et/ou une double coupure et/ou une coupure en trois positions. Ceci se produit notamment dans le cas où $R_1 = R_2 = R_3$ = OTr (possibilité d'obtention d'une monocoupure, d'une double coupure et d'une triple coupure), dans le cas où $R_1 = R_2 = R_3$ = OTBDMS (possibilité d'obtention d'une monocoupure et d'une double coupure) et dans le cas où $R_1 = R_3$ = OTBDMS et $R_2$ = OH (possibilité d'obtention d'une double et d'une triple coupure). L'homme de l'art comprendra que dans de tels cas, le choix des conditions opératoires les plus "dures" favorisera la triple coupure, la double coupure étant obtenue dans des conditions plus douces et la monocoupure, quand elle est possible, dans des conditions plus douces encore. L'homme de l'art déterminera aisément, dans les cas illustrés ci-dessus, la valeur des paramètres réactionnels (rapport molaire de l'agent d'oxydation au dérivé substitué du saccharose de départ, température, temps) qui permettent d'obtenir le cas échéant une coupure en une, deux ou trois positions.
On doit comprendre que les choix indiqués ci-dessus des groupements $R_1$, $R_2$ et $R_3$ permettant une régiosélectivité de la coupure oxydante en une, deux ou trois positions ne constituent que des exemples des possibilités du procédé de la présente invention et que tout autre choix desdits groupements $R_1$, $R_2$ et $R_3$ qui pourra être effectué à cet effet par l'homme de l'art se trouve dans le champ d'application de l'invention. Les produits de l'invention trouvent de nombreuses applications, notamment comme agents de réticulation, intermédiaires de synthèse et produits à propriétés bactériologiques.
L'invention sera décrite plus en détails dans les exemples non limitatifs suivants.

## Exemples

Les Exemples 1 et 2 décrivent une monocoupure oxydante en milieu biphasique, respectivement sans et avec catalyseur de transfert de phase. Les Exemples 3 et 4 décrivent une double coupure en milieu biphasique, l'Exemple 4 donnant en outre le mode de préparation d'un dérivé substitué du saccharose utilisé comme produit de départ qui est un produit nouveau. Les Exemples 5 et 6 décrivent une triple coupure oxydante, respectivement en solution aqueuse avec du métaperiodate de sodium et avec du tétraacétate de plomb. Les Exemples 7 et 8 décrivent un procédé de préparation de produits d'oxydation de saccharose substitué dont on a testé l'application comme produits bactériostatiques dans les Exemples 9 et 10.

### Exemple 1 : Oxydation periodique avec monocoupure en milieu biphasique du tri-O-t-butyldiméthylsily-1′,6,6′-saccharose sans catalyseur de transfert de phase

Une solution de tri-O-t-butyldiméthylsilyl-1′,6,6′-saccharose (voir FRANKE F., GUTHRIE R. D., Aust. J. Chem., 1977, 30, 639) (1 g) dans le chloroforme (20ml) est ajoutée à une solution aqueuse (20 ml) de métaperiodate de sodium (3,1 g) maintenue à 10°C. Après 12 H d'agitation, en émulsion et à température ambiante, la phase chloroformique est évaporée. Après réduction (butanol-1) et acétylation le sirop est chromatographié pour obtenir le produit ($R_1 = R_2 = R_3$ = OTBDMS ; $R_4$ = CHO ; $R_5$ = HOCH-CHO ; $R_6$-$R_7$ = CHOH-CHOH (configuration fructose) réduit et acétylé, $|\alpha|^{21}_{D}$-21,1 ($CHCl_3$, 0,8), Rf 0,4 (hexane-acétate d'éthyle 3:1).

R.m.n -$^1$H (300MHz ; $CDCl_3$) : δ/TMS 5,42(d, 1H, J3′, 4′ 5,5Hz, H-3′), 5,36(t,1H,J4′,5′ 6,0Hz,H-4′), 5,26(d, 1H, J1, 2 3,4Hz, H-1), 5,12-5,09(m, 1H), 4,36(m,1H), 4,16-4,03(m,5H), 3,78-3,65(m,6H).

R.m.n-$^{13}$C (75MHz; $CDCl_3$): δ/TMS 103,43(C-2′), 94,68(C-1), 80,96-74, 73-72,34(C-4,5,5′), 77,04(C-3′), 76,38(C-4′), 65,69-63,87-63,60-62,24-62,00(C-2,3,6,1′,6′).

Anal. Calc. pour $C_{40} H_{76} O_{16} Si_3$ C : 53,54 H : 8,54
trouvé C : 53,79 H : 8,80

**Exemple 2 : Oxydation periodique avec monocoupure en milieu biphasique du tri-O-trityl-1′,6,6′-saccharose en présence d'un catalyseur de transfert de phase**

A une solution de tri-O-trityl-1′,6,6′-saccharose (voir HOLZAPFEL C. W., KOEKEMOER J. M., MARAUS C. F., S. Afr. Tydskr. Chem., 1984,37, 57) (2,98 g ) dans du chloroforme (50 ml) on ajoute de l'Aliquat 336 (1,2 g) ainsi que le métaperiodate de sodium (4,4 g). Après 12 H d'agitation à température ambiante, filtration et évaporation, l'amine quaternaire est éliminée par chromatographie (hexane-acétate d'éthyle-triéthylamine 33:65:2). Après réduction (butanol-1) et acétylation, la séparation chromatographique conduit au produit ($R_1$ = $R_2$ = $R_3$ = OTr ; $R_4$ = HOCH-CHO ; $R_5$ = CHO ; $R_6$-$R_7$ = CHOH-CHOH (configuration fructose) réduit et acétylé, p.f. 59-60°C (éthanol), $|\alpha|_D^{21}$-17,0 ($CHCl_3$, 1,0 g), Rf 0,4 (toluène-acétone-triéthylamine 90:9:1).

R.m.n-$^1$H (300 Mhz ; $CDCl_3$) : δ/TMS 5,68 (d, 1H, J3′, 4′6, OHz, H-3′), 5,29-5,20 (m,2H,H-5,4′), 4,92 (dd, 1H, H-1), 4,42(dd, 1H, J6a,5 6,5Hz, H-6a), 4,13(m,1H,H-5′), 4,06-3,97(m,1H,H-4), 3,99(dd, 1H J6b, 6a 12Hz, H-6b), 3,86(dd,1H J2a,1 4, 7Hz, H-2a), 3,80(dd,1H,J2b,1 5,6Hz, J2b,2a 11,2Hz,H-2b), 3,39(dd,1H, J6′a,5′6,5Hz,H-6′a), 3,30(d,1H,H-1′-a),3,41-3,29(m,2H,H-3a,3b),3,18 (d,1H,J1′b,1′a 9,0Hz,H-1′b), 3,03(dd, 1H,J6′b,5 4,3Hz,J6′b,6′a 10,3Hz, H-6′b).

R.m.n-$^{13}$C (75MHz; $CDCl_3$) : δ/TMS 102,99(C-2′), 93,27(C-1), 78,83-77,39-76,39-72,28-70,10(C-4,5,3′,4′,5′), 66,32-64,96-64,72-62,84,60,24 (C-2,3,6,1′,6′). S.m. (ZAB-HF : α -hydroxy-4-nitrotoluène) m/z 1303(M+Na+).

Anal. Calc. pour $C_{79}H_{77}O_{16}$ C: 73.99; H:6,05.
Trouvé C: 73.77; H:6,10.

**Exemple 3 : Oxydation periodique du di-O-trityl-6,6′-saccharose avec double coupure en milieu biphasique.**

Une solution de di-O-trityl-6,6′-saccharose (voir HOUGH L., MUFTI K. S., KHAN R., Carbohydr. Res., 1972, 21, 144) (0,5 g) dans du chloroforme (20ml) est maintenue à 10°C pendant l'addition d'une solution aqueuse (20ml) de métaperiodate de sodium (1,4 g). Après 14 H d'agitation à température ambiante, la phase chloroformique est évaporée. Après réduction (butanol-1) et acétylation, on isole par chromatographie le produit ($R_1$ = $R_3$ = OTr ; $R_2$ = OH ; $R_4$ = $R_6$ = CHO ; $R_6$-$R_7$ = CHOH-CHOH (configuration fructose)) réduit et acétylé, p.f. 58°C (isopropanol), $|\alpha|_D^{21}$-39,5 ($CHCl_3$, 0,8), Rf 0,7 (hexane-acétate d'éthyle 1:1).

R.m.n-$^1$H (300 MHz, $CDCl_3$): δ/TMS 5,45(t,1H,J4′,5′ 6,0Hz, H-4′), 5,37(d,1H,J3′4′ 5,6Hz,H-3′), 5,23(t,1H,J1,2 5,2Hz, H-1), 4,33(d,1H,J1′a,1′b 12Hz, H-1′a), 4,14(d,1H,H-1′b), 4,16-4,10 (m,2H, H-1′b,5′), 4,05(q,1H,J2a,2b 11,8Hz,H-2a), 4,00-3,92 (m,3H,H-2b, 4a,4b), 3,78-3,75(m,1H,H-5), 3,38-3,34(m,1H,J6′a,6′b 15,0Hz,J6′a,5′ 5,6Hz, H-6′a), 3,26-3,22(m,1H,H-6′b), 3,04-3,05 (m,2H,H-6a,6b).

R.m.n-$^{13}$C (75MHz, $CDCl_3$): δ/TMS 102,47(C-2′), 95,04(C-1), 79,31-76,47-76,10-73,50(C-5,3′,4′,5′), 64,82-64,00-63,88-62,75 (C-2,4,6,1′,6′).

Anal. Calc. pour $C_{59}H_{60}O_{15}$: C: 70,22; H: 5,99
Trouvé : C: 70,11; H: 6,04

Exemple 4 : Oxydation periodique du mono-O-t-butyldiméthylsilyl-1′-**dichloro-6,6′-didésoxy-saccharose avec double coupure en milieu biphasique**

a) Synthèse du mono-O-t-butyldiméthylsily-1′-dichloro-6,6′-didésoxy-6,6′-saccharose

Une solution de dichloro-6-,6′-didésoxy-6,6′-saccharose (voir CHEN C.C., WHISTLER R. L., DANIEL J. R, Carbohydr. Res., 1983, 117, 318) (0,22 g) dans la pyridine (10ml) est agitée 12 H en présence de chlorure de t-butyldiméthylsilyle (0,32 g) à température ambiante. Après addition d'eau distillée glacée (100 ml) et agitation 30 mn, la solution est extraite à l'éther (3 x 10 ml). Après évaporation de la phase organique, le sirop est chromatographié pour donner le mono-O-t-butyldiméthylsily-1′-dichloro-6,6′-didésoxy-6,6′-saccharose (0,26g; 32%), p.f. 58°C, $|\alpha|^{21}_{D}$+47,5($CHCl_3$, 1,0), Rf 0,3 (éthanol-acétate d'éthyle-eau 4:54:8).

Anal. Calc. pour $C_{18}H_{34}Cl_2O_9Si$: C: 43,81; H: 6,95; Cl: 14,37
Trouvé C: 43,55; H: 7,05; Cl: 14,46

b) Caractérisation du mono-O-t-butyldiméthylsilyl-1′-dichloro-6,6′-didésoxy-6,6′-saccharose par l'intermédiaire de son polyacétate

L'acétylation (pyridine-anydride acétique) du mono-O-t-butyldiméthylsily-1′-dichloro-6,6′-didésoxy-6,6′-saccharose (0,2g) conduit après séparation chromatographique au penta-O-acétyl-2,3,4,3′-4′-mono-O-t-butyldiméthylsilyl-1′-dichloro-6,6′-didésoxy-6,6′-saccharose (0,16g ; 56%), p.f. 40°C, $|\alpha|^{21}_{D}$+53,8 ($CHCl_3$, 0,7) Rf 0,22 (hexane-acétate d'éthyle 7:3).

Anal. Calc. pour $C_{28}H_{44}Cl_2O_{14}Si$: C: 47,80; H: 6,30
Trouvé : C: 47,71; H: 6,25

c) Oxydation du mono-O-t-butyldimétylsilyl-1′-dichloro-6,6′-didésoxy-6,6′-saccharose

Une solution du mono-O-t-butyldimétylsilyl-1′-dichloro-6,6′-didésoxy-6,6′-saccharose (0,22 g) dans le chloroforme (5 ml) est ajoutée à une solution aqueuse (10 ml) de métaperiodate de sodium (0,37 g) maintenue à 10°C. Après agitation 12 H à température ambiante, la phase chloroformique est évaporée. Après réduction (butanol-1) et acétylation, la séparation chromatographique fournit le produit ($R_1$ = $R_3$ = Cl ; $R_2$ = OTBDMS ; $R_4$ = $R_5$ = CHO ; $R_5$-$R_7$ = CHOH-CHOH (configuration fructose)) réduit et acétylé, $|\alpha|^{21}_{D}$-31,8($CHCl_3$; 0,75), Rf 0,4 (hexane-acétate d'éthyle 3:1).

R.m.n-$^{1}$H (300 MHz, $CDCl_3$): δ/TMS 5,52(d, 1H,J3′,4′ 5,3Hz,H-3′), 5,35(q,1H), 5,30(t,1H,J4′,5′ 5,8Hz, H-4′), 4,45-3,58(m,12H).

R.m.n-$^{13}$C (75MHz; $CDCl_3$): δ/TMS 104,07(C-2′), 94,67(C-1), 80,41-76,83-76,53-72,96(C-5,3′,4′,5′), 65,22-64,66-63,82(C-2,4,1′), 44,28-43,48(C-6,6′)

Anal. Calc. pour $C_{25}H_{42}Cl_2O_{12}Si$: C: 47,39; H: 6,68
Trouvé : C: 47,21; H: 6,69.

**Exemple 5 : Oxydation periodique avec triple coupure du trichloro-6,1′,6′-tridésoxy-6,1′,6′-saccharose**

Une solution aqueuse (4 ml) de trichloro-6,1′,6′-tridésoxy-6,1′,6′-saccharose (voir HOUGH L., PHADNIS S. P., TARELLI E., Carbohydr. Res., 1975, 44, 12) (0,1 g) est amenée à 10°C avant d'ajouter le métaperiodate de sodium (0,3 g). Après 12 H d'agitation à température ambiante, puis neutralisation (NaHCO3), la précipitation des sels inorganiques est effectuée par addition d'éthanol (5 ml x 3)suivie de filtrations et d'évaporations. La réduction est faite dans un mélange eau-éthanol 1:1 (4 ml) avant d'acétyler. Après séparation chromatographique on obtient le produit ($R_1$ = $R_2$ = $R_3$ = Cl ; $R_4$ = $R_5$ = $R_5$ = $R_7$ = CHO) réduit et acétylé, $|\alpha|^{21}_{D}$ +2,6 ($CHCl_3$; 1,1), Rf 0,2(éther éthylique-éther de pétrole (40-60°C) 2:1).

R.m.n-$^{1}$H (300MHz; $CDCl_3$): δ/TMS 5,42(dd,1H), 4,39-4,17(m,8H), 4,23(dd,1H,H-2a), 4,14(dd,1H,J2,1 5,8Hz,J2a,2b 11,8Hz, H-2b), 3,77-3,63(m,6H,H-6a,6b,1′a,1′b,6′a,6′b).

R.m.n-$^{13}$C (300MHz; $CDCl_3$): δ/TMS 101,23(C-2′),94,01(C-1), 73,21-70,41 (C-5,5′), 64,08-63,66-63,16-62,85(C-2,4,3′,4′), 43,65-43,16(C-6,1′,6′).

| | | |
|---|---|---|
| Anal. Calc. pur $C_{19}H_{29}Cl_3O_{11}$: | C: 42,28; | H: 5,42 |
| Trouvé | C: 42,53; | H: 5,65 |

**Exemple 6 : Oxydation avec triple coupure du tri-O-trityl-1′,6,6′-saccharose à l'aide de $Pb(OAc)_4$**

Une solution chloroformique (20 ml) de tri-O-trityl-1′,6,6′-saccharose (1g) est agitée 12H à température ambiante en présence de $Pb(OAc)_4$ (1,62 g). Après addition d'acide oxalique (1,5 g), filtration et lavage par une solution saturée de NaHCO3 (3 x 100 ml) ainsi que d'un lavage à l'eau distillée (3 x 100 ml), la phase organique est évaporée. Après réduction (butanol-1) et acétylation la séparation chromatographique sur silice fournit le produit ($R_1 = R_2 = R_3$ = OTr; $R_4 = R_5 = R_5 = R_7$ = CHO) réduit et acétylé (0,58g; 51%), p.f. 70°C(éthanol), $|\alpha|^{21}_{D}$-8,4($CHCl_3$; 1,0), Rf 0,6 (hexane-acétate d'éthyle 2:1 avec 1% de $N(C_2H_5)_3$.

R.m.n-$^1$H (300MHz; CDCl3): δ/TMS 5,03(t,1H,J1,2 4,9Hz,H-1), 4,46(d,1H), 4,34-4,14 (m,4H), 4,01(d,2H), 3,93(dd,1H,J1,2 5,1Hz, J2a,2b 11,6Hz, H-2a), 3,85(dd,1H,J1,2 4,8Hz,J2a,2b 11,6Hz, H-2b), 3,47-3,32(m,3H), 3,12-3,06(m,4H).

R.m.N-$^{13}$C (75MHz; $CDCl_3$): δ/TMS 101,00(C-2′), 95,84(C-1), 74,53-69,18 (C-5,5′), 64,96-63,87-63,13-62,67-62,21-61,74 (C-2,4,6,1′,3′,4′,6′).

| | | |
|---|---|---|
| Anal. Calc. pour $C_{76}H_{74}O_{14}$ : | C: 75,41; | H: 6,20 |
| Trouvé | C: 75,31; | H: 6,02 |

**Exemple 7 : Préparation du dipalmitate-6-6′-saccharose oxydé par oxydation periodique**

On a effectué l'oxydation periodique du dipalmitate-6,6′-saccharose. Le mélange de dipalmitate-6-6′-saccharose (voir S. BOTTLE et I.D. JENKINS, J. Chem. Soc. Commun., 1984,385) (0,72 g), de chloloforme (30 ml) et d'eau déminéralisée (30 ml) a conduit à une émulsion dans laquelle on a ajouté du métaperiodate de Na (1,9 g) à 10°C. On a agité le mélange réactionnel pendant 1 H à 10°C et on a poursuivi l'agitation à température ambiante pendant 12H. Après évaporation partielle du chloroforme, filtration et lavage à l'eau, le précipité est séché (0,65 g).

On a obtenu du dipalmitate-6,6′-saccharose oxydé dont on a testé les propriétés bactériostatiques dans l'Exemple 9 ci-après.

**Exemple 8 : Préparation d'esters gras de saccharose commerciaux oxydés par oxydation periodique**

On a effectué l'oxydation periodique des deux mélanges suivants d'esters gras de saccharose provenant de la Société GATTEFOSSE :
8a : mono- et distéarate de saccharose (CAS 25168-73-4-E473)
8b : monopalmitate de saccharose (CAS 25168-73-4-E473)
en ajoutant une solution chloroformique (30 ml d'acide gras) de saccharose (2g), une solution aqueuse de métaperiodate de sodium (7,4g) et en agitant 12 H à température ambiante. Après évaporation du chloroforme, filtration, lavages du précipité à l'eau déminéralisée (10 x 20 ml), le précipité est séché (1,3-1,9 g). Taux en sodium ≦ 0,1 - 0,4%.
On a obtenu les produits 8a oxydé (8a-ox) et 8b oxydé (8b-ox) dont on a testé, dans l'Exemple 10 ci-après, les propriétés bactériostatiques en les comparant aux produits non oxydés 8a et 8b.

**Exemple 9 : Propriétés bactériostatiques du produit de l'Exemple 7**

On a préparé des mélanges de solutions limpides de l'échantillon et du milieu de culture, contenant un adjuvant de dissolution, ajouté à raison de la quantité minimale nécessaire.

On a introduit les solutions obtenues telles quelles ou diluées dans des tubes stériles sous un volume inférieur ou égal à 2,5 ml, que l'on a ensuite ajusté sinécessaire à 2,5 ml à l'aide d'eau stérile. On a alors ajouté à chaque tube 2,5 ml de bouillon nutritif préparé à concentration double, ensemencé de façon à contenir environ

1000 germes par ml et ayant la composition suivante :

| | |
|---|---|
| peptone bactériologique | 10 g |
| extrait de viande de boeuf | 5 g |
| chlorure de sodium | 5 g |
| eau déminéralisèe Q S P | 500 ml |

pH ajusté à 7,2 à l'aide de potasse.
stérilisation par fractions de 50 ml 20 mn à 120°C

Les souches testées sont cultivées sur milieu gélosé "trypticase-soja" dont la compositon de formulation :

| | |
|---|---|
| peptone trypsique de caséine | 15 g |
| peptone papaïnique de soja | 5 g |
| chlorure de sodium | 5 g |
| gélose | 15 g |
| eau déminéralisée Q S P | 1000 ml |

pH ajusté à l'aide de potasse à 7,3
stérilisation 20 mn à 120°C.

Après incubation 18 H à 35°C on ensemence à l'aide de ces cultures des tubes de milieu liquide "trypticase-soja" de formulation :

| | |
|---|---|
| peptone trypsique de caséine | 17 g |
| peptone papaïnique de soja | 3 g |
| chlorure de sodium | 5 g |
| phosphate dipotassique | 2,5 g |
| glucose | 2,5 g |
| eau déminéralisée Q S P | 1000 ml |

pH ajusté à 7,3 à l'aide de potasse
stérilisation 20 mn à 120°C.

Après 18 H d'incubation à 35°C, les cultures obtenues sont homogénéisées puis diluées.

Les quatres souches suivantes ont été testées :

– Streptoccus fecalis (ATCC 9790)
– Escherichia coli (ATCC 10536)
– Proteus morganii (ATCC 53185)
– Staphylococcus (ATCC 6538P)

On a effectué trois essais, en utilisant les gammes de concentrations suivantes de dipalmitate-6,6'-saccharose oxydé

| Essai | Concentration (mg/ml) |
|---|---|
| N° 1 | 0,1 - 1 - 10 |
| N° 2 | 0,1 - 0,2 - 0,4<br>0,8 - 1,6 |
| N°3 | 0,006 - 0,012 - 0,025<br>0,05 - 0,1 - 0,2<br>0,4 - 0,8 - 1,6 - 3,2 |

On a effectué l'incubation des tubes à 35°C. Les lectures ont été faites après 18, 24 et 48 H en comparaison avec des tubes témoins de croissance de chaque microorganisme testé, en présence d'un agent tensio-actif : Tween 80 (marque de fabrique d'Atlas Chemical Industries, Inc) pour l'essai N° 1 et Triton X 100, (marque de fabrique de Rohm & Haas Co) pour les essais N°2 et N°3.

Les résultats, donnés dans le Tableau I ci-dessous, relevés après 24 H d'incubation, indiquent la concentration (mg/ml) testée en dipalmitate-6,6'-saccharose oxydé à partir de laquelle on observe une absence de développement bactérien pour chacune des souches étudiées.

TABLEAU I

| Essai | Staphyloccus ATCC 6538 P | Streptoccus fecalis ATCC 9790 | Escherichia coli ATCC 10536 | Proteus morganii ATCC 53185 |
|---|---|---|---|---|
| N°1 | - | 1 | 1 | 1 |
| N°2 | < 0,1 | 0,8 | 0,8 | 0,2 |
| N°3 | 0,01 | 0,8 | 1,6 | 0,2 |

Il résulte de cette recherche de la concentration minimale inhibitrice (CMI), effectuée par la technique de dilution en milieu liquide mise en oeuvre, que le dipalmitate-6,6'-saccharose oxydé possède une bonne activité bactériostatique à une CMI de 1,6 mg/ml. Le staphylococcus (ATCC 6538 P) est particulièrement sensible à ce produit, une concentration d'au plus 0,01 mg/ml étant suffisante pour inhiber son développement.

**Exemple 10 : Propriétés bactériostatiques des produits de l'Exemple 8**

On a testé les propriétés bactériostatiques des produits de l'Exemple 8 que l'on a comparées à leurs dérivés esters gras du saccharose de départ sur les souches suivantes :
Staphylococcus (ATCC 6538 P)
Escherichia coli (ATCC 10536)
Proteus morganii (ATCC 53185)
cultivées comme dans l'Exemple 9.

On a effectué l'incubation des tubes à 35°C ± 1°C. Les lectures ont été effectuées après 20, 24 et 48 H en comparaison avec des tubes témoin de croissance de chaque microorganisme testé, sans Triton X 100 dans le premier essai et avec du Triton X 100 dans le deuxième essai.

Les résultats, donnés dans le Tableau II ci-dessous, relevés après 48 H d'incubation (les cultures ont évolué entre la 24ème et la 48ème heure pour les produits testés), donnent pour chacun des produits testés : 8a, 8a-ox, 8b, 8b-ox, la concentration à partir de laquelle on observe une absence de développement bactérien pour chacune des souches étudiées.

## TABLEAU II

| | 8a | 8a-ox | 8-b | 8b-ox |
|---|---|---|---|---|
| 1er essai | | | | |
| Staphylococcus ATCC 6528 P | > 0,6 | ≈ 0,125 | > 0,6 | 0,05 |
| Escherichia coli ATCC 10536 | > 1,25 | ≈ 0,6 | > 1,25 | 0,125 |
| Proteus morganii ATCC 53185 | > 1,25 | < 0,125 | > 0,6 | < 0,12 |
| 2ème essai | | | | |
| Staphyloccus ATCC 6538 P | 1,3 | ≈ 0,08 | 1,2 | ≈ 0,02 |
| Escherichia coli ATCC 10536 | > 2 | 0,35 | 1,25 | ≈ 0,12 |
| Proteus morganii ATCC 53185 | 1,5 | 0,2 | 1,5 | ≈ 0,3 |

Les résultats du Tableau II montrent que les produits 8a-ox et 8b-ox de la présente invention agissent en quantité beaucoup plus faible (respectivement facteur 10 et facteur 5-10) que les produits de l'art antérieur 8a et 8b comme agents bactériostatiques vis-à-vis des souches étudiées.

### Revendications

1. Nouveaux produits dérivés du saccharose représentés par la formule générale (I)

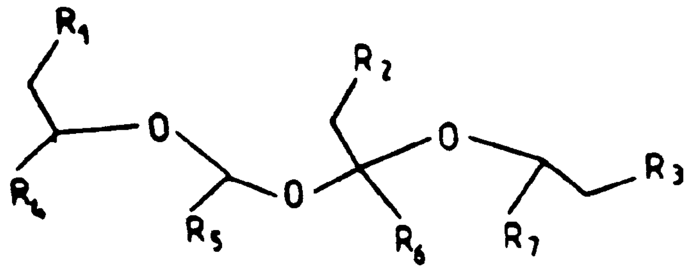

(I)

dans laquelle :

(a) Les groupements $R_1$, $R_2$ et $R_3$, sont choisis parmi les radicaux X, OH, OR et OCOR′, où X est F, Cl, Br ou I, R est un groupement hydrocarboné contenant de 1 à 30 atomes de carbone et de 0 à 10 atomes de silicium et R′ est un groupement hydrocarboné contenant de 1 à 30 atomes de carbone,

(b) Les groupements $R_4$ et $R_5$, identiques ou différents, sont choisis parmi les radicaux CHO, HOCH-CHO, ou bien $R_4$-$R_5$ = CHOH-CHOH-CHOH (configuration glucose),

(c) Les groupements $R_6$ et $R_7$ sont CHO ou bien $R_6$-$R_7$ = CHOH-CHOH (configuration fructose), et dans laquelle

(i) on n'a pas en même temps

$R_4$-$R_6$ = CHOH - CHOH- CHOH (configuration glucose) et

$R_6$-$R_7$ = CHOH - CHOH (configuration fructose)

et

(ii) on n'a pas en même temps $R_1$ = $R_2$ = $R_3$ = OH.

2. Produits selon la revendication 1 dans lesquels ledit groupement R est choisi parmi les groupements alkyle, alkylaryle, aryle, alkylsilyle, arylsilyle et alkylarylsilyle contenant jusqu'à 30 atomes de carbone et jusqu'à 10 atomes de silicium et R′ est choisi parmi les groupements alkyle, alcényle et alcynyle contenant jusqu'à 30 atomes de carbone.

3. Produits selon la revendication 2 dans lesquels X est Cl, R est choisi parmi les radicaux Tr, TBDMS et TBDPS où

– Tr représente le radical :

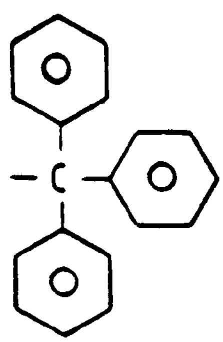

– TBDMS représente le radical tert-butyldiméthylsilyle de formule :

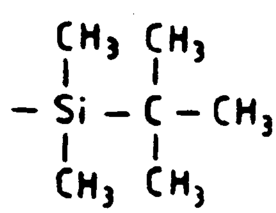

– TBDPS représente le radical tert-butyldiphénylsilyle de formule :

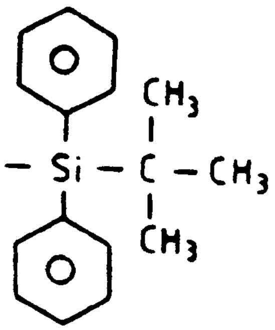

et R′ est choisi parmi les groupements $C_nH_{2n+1}$ où n est un nombre entier pouvant aller de 1 à 30.

4. Procédé de préparation des produits selon l'une des revendications 1 à 3 caractérisé par le fait que l'on effectue une coupure oxydante d'un dérivé substitué du saccharose représenté par la formule générale (II)

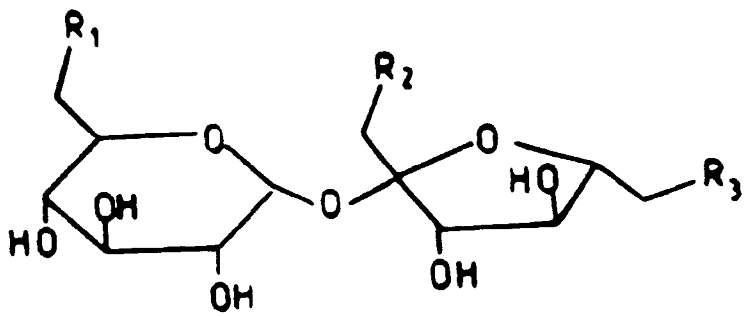

(II)

dans laquelle $R_1$, $R_2$ et $R_3$ sont définis comme dans la revendication 1 en présence d'un agent d'oxydation choisi parmi les produits susceptibles de libérer l'ion periodate et le tétraacétate de plomb.

5. Procédé selon la revendication 4 dans lequel ledit agent d'oxydation est un produit susceptible de libérer l'ion periodate.

6. Procédé selon la revendication 4 dans lequel ledit agent d'oxydation est le tétraacétate de plomb.

7. Procédé selon la revendication 5 dans lequel ledit produit susceptible de libérer l'ion periodate est choisi parmi l'acide periodique et ses sels et les periodates fixés sur résine, alumine ou silice.

8. Procédé selon l'une des revendication 5 et 7 dans lequel on dissout ledit dérivé substitué du saccharose dans un solvant approprié et que l'on ajoute à la solution résultante une solution aqueuse contenant l'ion periodate en quantité telle que le rapport molaire du periodate dudit dérivé substitué du saccharose soit compris entre 1 et 50, de préférence entre 4 et 20.

9. Procédé selon la revendication 8 dans lequel ledit solvant est choisi parmi l'eau et les solvants organiques.

10. Procédé selon la revendication 9 dans lequel ledit solvant organique est choisi parmi le chloroforme et le dichlorométhane.

11. Procédé selon l'une des revendications 7 à 10 dans lequel on incorpore un agent de transfert de phase.

12. Procédé selon l'une des revendications 7 à 11 dans lequel on maintient d'abord le mélange réactionnel à une température comprise entre -20 et 80°C, de préférence entre 0 et 20°C, et que l'on laisse la réaction se poursuivre pendant une durée suffisante, comprise entre environ 2 et 48 Heures.

13. Procédé selon la revendication 6 dans lequel le rapport molaire du tétraacétate de plomb, au dérivé substitué du saccharose de départ est au moins égal à 1, de préférence au moins égal à 4.

14. Procédé selon l'une des revendications 6 et 13 dans lequel de dérivé substitué du saccharose de départ est dissous dans un solvant organique approprié.

15. Procédé selon la revendication 14 dans lequel ledit solvant organique est choisi parmi le chloroforme et la pyridine.

16. Procédé selon l'une des revendications 6, 14 et 15 dans lequel on maintient le mélange réactionnel à une température comprise entre -20 et 80°C, de préférence à température ambiante pendant 2 à 48 heures.

17. Procédé selon la revendication 4 caractérisé par le fait que l'on choisit lesdits groupements $R_1$, $R_2$ et $R_3$ de façon appropriée pour permettre une régiosélectivité de ladite coupure oxydante en une, deux ou trois positions.

18. Procédé selon la revendication 17 caractérisé par le fait que les groupements $R_1$, $R_2$, $R_3$ sont égaux entre eux et choisis parmi OTr et OTBDMS, ledit procédé permettant une monocoupure régiosélective de l'unité glucopyranosidique du dérivé substitué du saccharose de départ, permettant d'orienter ladite coupure oxydante respectivement sur la liaison C-2, C-3 et C-3, C-4.

19. Procédé selon la revendication 17 caractérisé par le fait que $R_1$, $R_2$ et $R_3$ sont tels que :

$R_1 = R_3 = Cl$ et $R_2$ = OTBDMS ou OTr

ou

$R_1 = R_2 = R_3$ = OTBDMS, OTr ou OTBDPS

ou

$R_1 = R_3$= OTr ou OTBDMS et $R_2 = OH$

ou

$R_1$ = OTr et $R_2 = R_3 = OH$

permettant une double coupure sélective de l'unité glucopyranosidique du dérivé substitué du saccharose de départ.

20. Procédé selon la revendication 17, caractérisé par le fait que $R_1$, $R_2$, $R_3$ sont tels que :

$R_1 = R_2 = R_3 = Cl$

ou

$R_1 = R_2 = R_3$ = OTr

ou

$R_1 = R_3$ = OTBDMS et $R_2 = OH$

ou

$R_1 = R_3 = OCOC_nH_{2n+1}$ et $R_2 = OH$

ou

$R_1 = R_2 = R_3$ = OH ou $OCOC_nH_{2n}$+1

permettant une triple coupure au dérivé substitué du saccharose de départ en trois positions glycol-1,2.

21. Mono-<u>O</u>-t-butylméthylsilyl-1′-dichloro-6,6′-didésoxy-6,6′-saccharose de formule :

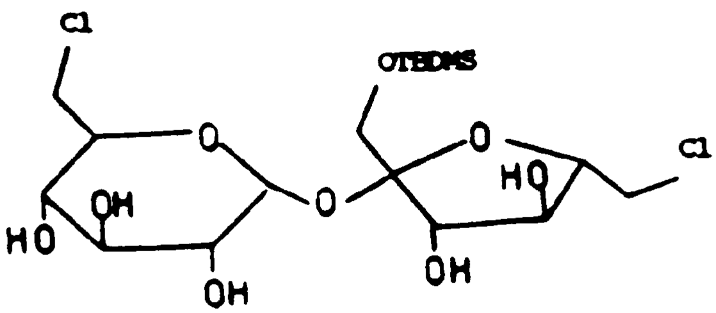

dans laquelle TBDMS est le radical de formule :

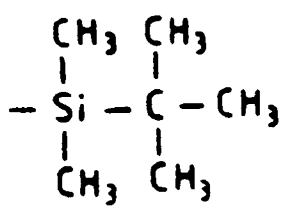

22. Préparation du produit de la revendication 21 consistant à faire réagir une solution de dichloro-6,6′-didésoxy-6,6′-saccharose dans la pyridine et du chlorure de t-butyldiméthylsilyle pendant au moins 12 H à température ambiante.

23. Application des produits selon l'une des revendications 1 à 3 comme agents bactériostatiques.

**Claims**

1. Novel products derived from saccharose and represented by the following general formula (I)

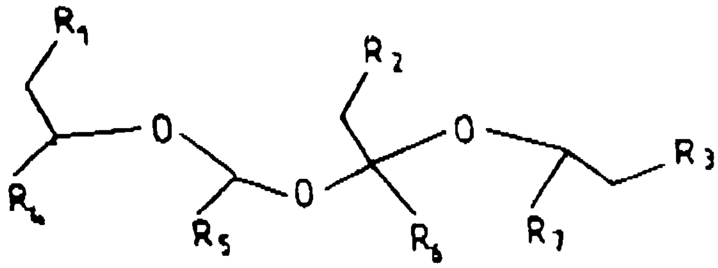

(I)

in which:

(a) The groups $R_1$, $R_2$ and $R_3$ are choses from among the radicals X, OH, OR and OCOR', where X is F, Cl, Br or I, R is a hydrocarbon group containing 1 to 30 carbon atoms and 0 to 10 silicon atoms and R' is a hydrocarbon group containing 1 to 30 carbon atoms,

(b) The groups $R_4$ and $R_5$ are identical or different and are chosen from among the radicals CHO, HOCH-CHO or $R_4$-$R_5$ = CHOH-CHOH-CHOH (glucose configuration),

(c) The groups $R_6$ and $R_7$ are CHO or $R_5$-$R_7$ = CHOH-CHOH (fructose configuration) and in which

(i) $R_4$-$R_5$ = CHOH - CHOH - CHOH (glucose configuration) does not occur simultaneously with $R_6$-$R_7$ = CHOH - CHOH (fructose configuration) and

(ii) $R_1$ = $R_2$ = $R_3$ = OH is not true simultaneously the case.

2. Products according to claim 1, in which the R group is chosen from among allyl, alkylaryl, aryl, alkyl silyl, aryl silyl and alkyl aryl silyl groups containing up to 30 carbon atoms and up to 10 silicon atoms and R' is chosen from among alkyl, alkenyl and alkynyl groups containing up to 30 carbos atoms.

3. Products according to claim 2, in which X is Cl, R is chosen from among the radicals Tr, TBDMS and TBDPS where

– Tr represents the radical:

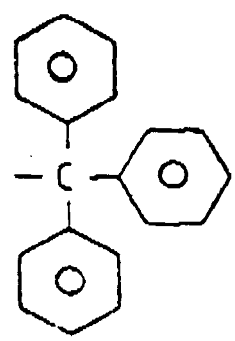

– TBDMS represents the tert.butyl dimethyl silyl radical having the formula:

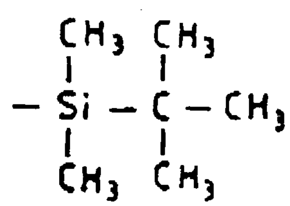

– TBDPS represents the tert.butyl diphenyl silyl radical having the formula:

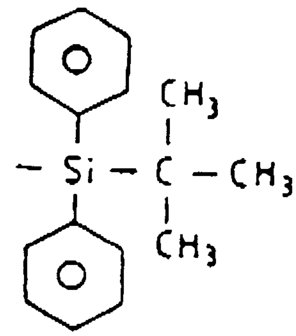

and R′ is chosen from among $c_nH_{2n+1n}$ groups where n is an integer from 1 ta 30.

4. A method of preparing products according to any of claims 1 to 3, characterised in that an oxidising cleavage is made of a substituted derivative of saccharose represented by the general formula (II)

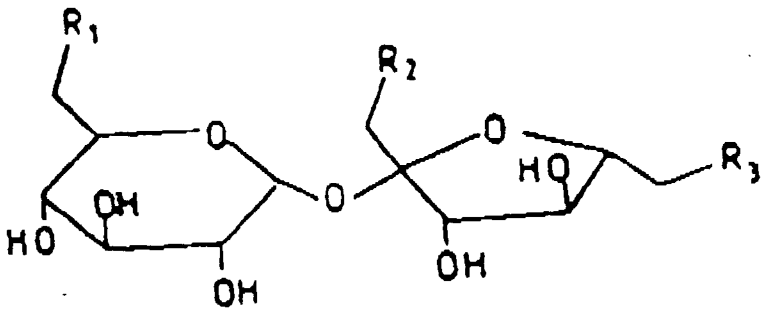

(II)

in which $R_1$, $R_2$ and $R_3$ are defined as in claim 1, in the presence of an oxidising agent chosen from among the products capable of releasing the periodate ion and lead tetra-acetate.

5. A method according to claim 4, in which the oxidising agent is a product capable of releasing the periodate ion.

6. A method according to claim 4, in which the oxidising agent is lead tetra-acetate.

7. A method according to claim 5, is which the product capable of releasing the periodate ion is chosen from among periodic acid and salts thereof and periodates fixed on resin, alumina or silica.

8. A method according to any of claims 5 to 7, in which the substituted derivative of saccharose is dissolved in a suitable solvent and an aqueous solution is added to the resulting solution, the aqueous solution containing the periodate ion in a quantity such that the molar ratio of periodate to the substituted derivative of saccharose is between 1 and 50, preferably between 4 and 20.

9. A method according to claim 8, in which the solvent is chosen from among water and organic solvents.

10. A method according to claim 9, in which the organic solvent is chosen from among chloroform and dichloromethase.

11. A method according to any of claims 7 to 10, in which a phase transfer agent is incorporated.

12. A method according to any of claims 7 to 11, in which the reaction mixture is initially maintained at a temperature between -20 and 80°C, preferably between 0 and 20°C, and the reaction is allowed to continue for a sufficient time, between about 2 and 48 hours.

13. A method according to claim 6, in which the molar ratio of lead tetra-acetate to the substituted derivative of the starting saccharose is at least equal to 1 and is preferably at least equal to 4.

14. A method according to claim 6 or claim 13, in which the substituted derivative of the starting saccharose is dissolved in a suitable organic solvent.

15. A method according to claim 14, is which the organic solvent is chosen from among chloroform and pyridise.

16. A method according to claim 6 or 14 or 15, in which the reaction mixture is maintained at a temperature between -20 and 80°C, preferably at ambient temperature, for 2 to 48 hours.

17. A method according to claim 4, characterised in that the groups $R_1$, $R_2$ and $R_3$ are suitably chosen so as to obtain regional selectivity of the oxidising cleavage at one, two or three positions.

18. A method according to claim 17, characterised in that the groups $R_1$, $R_2$ and $R_3$ are identical and are chosen from among OTr asd OTBDMS, the method being used for regionally selective mono-cleavage of the glucopyranoside unit of the substituted starting saccharose derivative, so as to orient the oxidising cleavage along the C-2, C-3 and C-3, C-4 bond respectively.

19. A method according to claim 17, characterised in that $R_1$, $R_2$ and $R_3$ are such that
$R_1$ = $R_3$ = Cl and $R_2$ = OTBDMS or OTr
or
$R_1$ = $R_2$ = $R_3$ = OTBDMS, OTr or OTBDPS
or
$R_1$ = $R_3$ = OTr or OTBDMS and $R_2$ = OH
or
$R_1$ = OTr and $R_2$ = $R_3$ = OH
so to obtain a double selective cleavage of the glucopyranoside unit of the substituted derivative of the starting saccharose.

20. A method according to claim 17, characterised in that $R_1$, $R_2$ and $R_3$ are such that
$R_1$ = $R_2$ = $R_3$ = Cl
or
$R_1$ = $R_2$ = $R_3$ = OTr
or
$R_1$ = $R_3$ = OTBDMS and $R_2$ = OH
or
$R_1$ = $R_3$ = $OCOC_nH_{2n+1}$ and $R_2$ = OH
or
$R_1$ = $R_2$ = $R_3$ = OH or $OCOC_nH_{2n}$+1
so as to obtain a triple cleavage at the substituted Derivative of the starting saccharose at three 1,2-glycol positions.

21. Mono-o-t-butyl methyl 1′-silyl 6,6′-dichloro 6,6′-dideoxy saccharose having the formula:

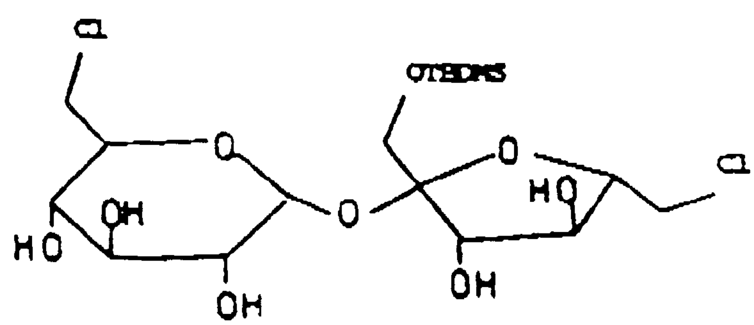

is which TBDMS is the radical having the formula:

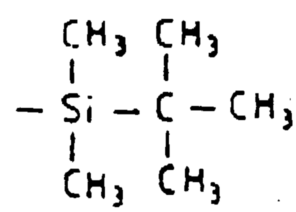

22. Preparation of the product in claim 21 by reacting a solution of 6,6′-dichloro 6,6′-dideoxy saccharose in pyridine and t-butyl dimethyl silyl chloride for at least 12 hours at ambient temperature.

23. Use of the products according to asy of claims 1 to 3 as bacteriostatic agents.

**Patentansprüche**

1.Neue, von Saccharose abstammende Stoffe mit der allgemeinen Formel (I)

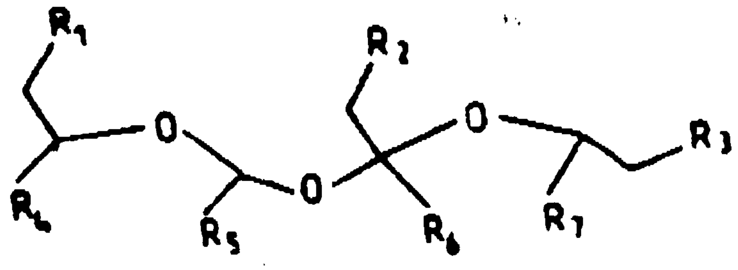

(I)

in der:

(a) die Reste $R_1$, $R_2$ und $R_3$ unter den Radikalen X, OH,OR und OCOR′ ausgewählt sind, wobei X für F, Cl, Br oder I steht, R eine Kohlenwasserstoffgruppe ist, die l bis 30 Kohlenstoffatome und 0 bis 10 Siliciumatome enthält, und R′ eine Kohlenwasserstoffgruppe mit 1 bis 30 Kohlenstoffatomen ist,

(b) die Reste $R_4$ und $R_5$, die identisch oder verschieden sind, unter den Radikalen CHO, HOCH-CHO ausgewählt sind, oder aber $R_4$-$R_5$ = CHOH-CHOH-CHOH (Glukose-Konfiguration),

(c) die Reste $RT_6$ und $R_7$ CHO sind, oder aber $R_5$-$R_7$ = CHOH-CHOH (Fructose-Konfiguration), und in der

(i) nicht gleichzeitig $R_4$-$R_5$ = CHOH - CHOH - CHOH (Glucose-Konfiguration) und $R_5$-$R_7$ = CHOH - CHOH (Fructose-Konfiguration) ist und

(ii) nicht gleichzeitig $R_1$ = $R_2$ = $R_3$ = OH ist.

2. Stoffe nach Anspruch 1, in denen der Rest R unter den Gruppen Alkyl, Alkylaryl, Aryl, Alkylsilyl, Arylsilyl und Alklarylsilyl ausgewählt ist, die bis zu 30 Kohlenstoffatome und bis zu 10 Siliciumatome enthalten, und R′ unter den Gruppen Alkyl, Alkenyl und Alkinyl, die bis zu 30 Kohlenstoffatome enthalten, ausgewählt ist.

3. Stoffe nach Anspruch 2, in denen X Cl ist, R unter den Radikalen Tr, TBDMS und TBDPS ausgewählt ist, worin

– Tr das Radikal darstellt:

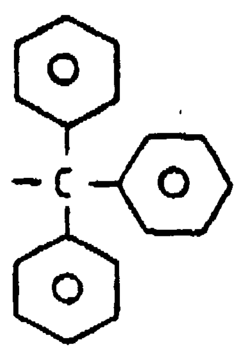

– TBDMS das Radikal Tert-butyldimethylsilyl darstellt mit der Formel:

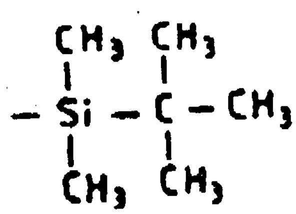

– TBDPS das Radikal, Tert-butyldiphenylsilyl darstellt mit der Formel:

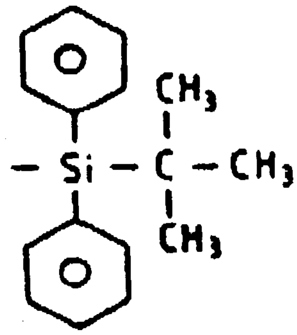

und R′ ausgewählt ist unter den Gruppen $C_nH_{2n+1}$, worin n eine ganze Zahl von 1 bis 30 sein kann.

4. Verfahren zur Herstellung der Stoffe nach einem der Ansprüche 1 bis 3, dadurch gekennzeichnet, daß eine oxidative Spaltung eines substituierten Saccharose-Derivats mit der allgemeinen Formel (II)

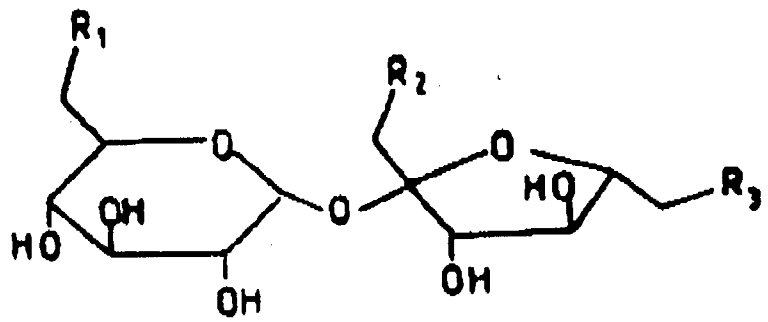

(II)

, in der $R_1$, $R_2$ und $R_3$ wie in Anspruch 1 definiert sind, in Gegenwart eines Oxidationsmittels, welches unter Stoffen ausgewählt ist, die ein Periodation freisetzen, sowie unter Bleitetraacetat, durchgeführt wird.

5. Verfahren nach Anspruch 4, bei dem das Oxidationsmittel ein Stoff ist, der ein Periodation freisetzt.

6. Verfahren nach Anspruch 4, bei dem das Oxidationsmittel Bleitetraacetat ist.

7. Verfahren nach Anspruch 5, bei dem der Stoff, der ein Periodation freisetzen kann, ausgewählt ist unter Periodsäure und ihren Salzen und Periodaten, die an Harz, Aluminium oder Kieselerde gebunden sind.

8. Verfahren nach einem der Ansprüche 5 und 7, bei dem das substituierte Saccharose-Derivat in einem geeigneten Lösungsmittel gelöst und eine Lösung hinzugefügt wird, so daß eine wässrige Lösung entsteht, die ein Periodation in solch ein Quantität enthält, daß das Molverhältnis von Periodat zu substituiertem Saccharose-Derivat zwischen 1 und 50 liegt, vorzugsweise zwischen 4 und 20.

9. Verfahren nach Anspruch 8, bei dem das Lösgungsmittel ausgewählt ist unter Wasser und organischen Lösgungsmitteln.

10. Verfahren nach Anspruch 9, bei dem das organische Lösungsmittel ausgewählt ist unter Chloroform und Dichlormethan.

11. Verfahren nach einem der Ansprüche 7 bis 10, bei dem ein Phasenübertragungsagens eingesetzt wird.

12. Verfahren nach einem der Ansprüche 7 bis 11, bei dem das Reaktionsgemisch auf einer Temperatur zwischen -20 und 80°C, vorzugsweise zwischen 0 und 20°C, gehalten wird und die Reaktion für einen ausreichenden Zeitraum, der etwa zwischen 2 und 48 Stunden liegt, weiterläuft.

13. Verfahren nach Anspruch 6, bei dem das Molverhältnis von Bleitetraacetat zu dem anfänglichen, substituierten Saccharose-Derivat wenigstens gleich 1 ist, vorzugsweise wenigstens gleich 4.

14. Verfahren nach einem der Ansprüche 6 und 13, bei dem das anfängliche, substituierte Saccharose-Derivat in einem geeigneten, organischen Lösungsmittel gelöst wird.

15. Verfahren nach Anspruch 14, bei dem das organische Lösungsmittel ausgewählt wird unter Chloroform und Pyridin.

16. Verfahren nach einem der Ansprüche 6, 14 und 15, bei dem das Reaktionsgemisch auf einer Temperatur zwischen -20 und 80°C, vorzugsweise auf Umgebungstemperatur, für 2 bis 48 Stunden gehalten wird.

17. Verfahren nach Anspruch 4, dadurch gekennzeichnet, daß die Reste $R_1$, $R_2$ und $R_3$ derart geeignet ausgewählt sind, daß sie eine Raumselektivität für eine oxidative Spaltung an einer, zwei oder drei Positionen erlauben.

18. Verfahren nach Anspruch 17, dadurch gekennzeichnet, daß die Reste $R_1$, $R_2$, $R_3$ gleich sind und unter

OTr und OTBDMS ausgewählt sind, das Verfahren eine raumselektive Einfachspaltung an der glucopyranosidischen Einheit des anfänglichen, substituierten Saccharose-Derivats erlaubt sowie eine Ausrichtung der oxidativen Spaltung ander Bindung C-2, C-3 bzw. C-3, C-4.

19. Verfahren nach Anspruch 17, dadurch gekennzeichnet, daß die Reste $R_1$, $R_2$ und $R_3$ wie folgt sind:
$R_1 = R_3 = Cl$ und $R_2$ = OTBDMS oder OTr
oder
$R_1 = R_2 = R_3$ = OTBDMS, OTr oder OTBDPS
oder
$R_1 = R_3$ = OTr oder OTBDMS und $R_2$ = OH
oder
$R_1$ = OTr und $R_2 = R_3$ = OH,
und daß eine selektive Doppelspaltung der glucopyranosidischen Einheit des anfänglichen, substituierten Saccharose-Derivats möglich ist.

20. Verfahren nach Anspruch 17, dadurch gekennzeichnet, daß $R_1$, $R_2$, $R_3$ wie folgt sind:
$R_1 = R_2 = R_3 = Cl$
oder
$R_1 = R_2 = R_3$ =OTr
oder
$R_1 = R_3$ = OTBDMS und $R_2$ = OH
oder
$R_1 = R_3 = OCOC_nH_{2n+1}$ und $R_2$ = OH
oder
$R_1 = R_2 = R_3$ = OH oder $OCOC_nH_{2n+1}$,
und daß eine Dreifachspaltung an dem anfänglichen substituierten Saccharosen-Derivat an drei Glycol-1,2 Positionen möglich ist.

21. Mono-O-t-butylmethylsilyl-1′-dichlor-6,6′-didesoxy-6,6′-saccharose mit der Formel:

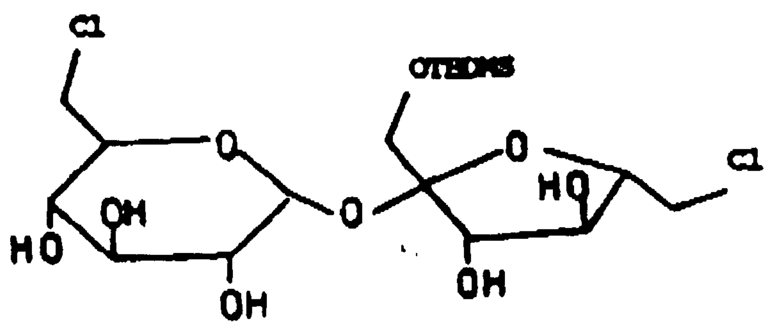

in der RBDMS ein Radikal der Formel:

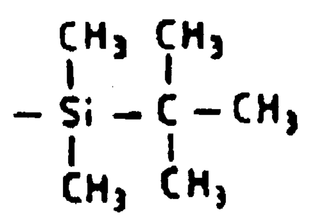

ist.

22. Herstellung eines Stoffes nach Anspruch 21, wobei eine Lösung von Dichlor-6,6′-didesoxy-6,6′-saccharose in Pyridin und von t-Butyldimethylsilylchlorid für wenigstens 12 Stunden bei Umgebungstemperatur reagiert.

23. Verwendung der Stoffe nach einem der Ansprüche 1 bis 3 als bakteriostatische Agentien.